(19) Europäisches Patentamt
European Patent Office
Office européen des brevets

(11) Veröffentlichungsnummer: **0 183 649**
**A1**

(12) # EUROPÄISCHE PATENTANMELDUNG

(21) Anmeldenummer: **85810555.4**

(22) Anmeldetag: **21.11.85**

(51) Int. Cl.[4]: **C 07 D 311/92**
A 61 K 31/35

(30) Priorität: **26.11.84 CH 5629/84**

(43) Veröffentlichungstag der Anmeldung:
**04.06.86 Patentblatt 86/23**

(84) Benannte Vertragsstaaten:
**AT BE CH DE FR GB IT LI LU NL SE**

(71) Anmelder: **CIBA-GEIGY AG**
**Klybeckstrasse 141**
**CH-4002 Basel(CH)**

(72) Erfinder: **Schaffner, Karl, Dr.**
**Im Goldbrunnen 37**
**CH-4104 Oberwil(CH)**

(72) Erfinder: **Traxler, Peter, Dr.**
**Bündtenring 5**
**CH-4124 Schönenbuch(CH)**

(72) Erfinder: **Zimmermann, Markus, Dr.**
**Steinbrecheweg 8**
**CH-4125 Riehen(CH)**

(72) Erfinder: **Wehrli, Walter, Dr.**
**Aescherstrasse 23**
**CH-4054 Basel(CH)**

(72) Erfinder: **Schmidtruppin, Karl Heinz, Dr.**
**Im Baumgarten 5**
**CH-4144 Arlesheim(CH)**

(54) **Stickstoffderivate von Oxoverbindungen.**

(57) Verbindungen der Formel

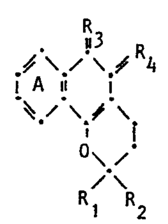

(I)

worin $R_1$ und $R_2$ unabhängig voneinander Wasserstoff, Niederalkyl oder Aryl oder zusammengenommen Niederalkylen bedeuten, einer der Reste $R_3$ und $R_4$ die Gruppe =N–R (Ia) darstellt, worin R die Gruppe –O–R–a (Ib) und $R_a$ Wasserstoff, Niederalkyl, Carboxyniederalkyl oder Niederalkoxycarbonylniederalkyl oder R die gruppe $-N(R_b)-R_c$ (Ic) und $R_b$ und $R_c$ unabhängig voneinander Wasserstoff oder Niederalkyl oder einer der Reste $R_b$ und $R_c$ Wasserstoff und der andere Rest die Gruppe $-C(=X)-R_d$ darstellt, worin X Oxo und $R_d$ Niederalkoxy, Diniederalkylaminoniederalkyl, Triniederalkylammonioniederalkyl, Nideralkylenaminoniederalkyl, Oxaniderakylenammonioniederalkyl, Thianiederalkylenaminoniederalkyl, gegebenenfalls durch Niederalkyl aza-substituiertes Anzaniederalkylenaminoniederalkyl oder Pyridinioniederalkyl oder X Oxo oder Thioxo und $R_d$ Amino bedeuten, oder beide Reste $R_b$ und $R_c$ zusammengenommen Niederalkylen, Oxaniederalkylen, Thianiederalkylen oder gegebenenfalls durch Niederalkyl aza-substituiertes Aznaniederalkylen bedeuten, und der andere der beiden Reste $R_3$ und $R_4$ dieselben Bedeutungen hat oder Oxo bedeutet und der Ring A keine weiteren Substituenten hat oder zusätzlich durch Niederalkyl, Niederalkoxy und/oder Halogen substituiert ist, sowei Salze von solchen Verbindungen mit salzbildenen Eigenschaften sind wirksam bei der durch einen Retrovirus induzierten Rauscher Leukämie der Maus und können daher therapeutisch beispielsweise bei der Bekämpfung von ebenfalls durch Retroviren verursachten Immunkrankheiten oder Autoimmunkrankheiten verwendet werden.

EP 0 183 649 A1

0183649

4-15163/+

## Stickstoffderivate von Oxoverbindungen

Die vorliegende Erfindung betrifft Stickstoffderivate von Oxoverbindungen sowie deren Herstellung, ferner pharmazeutische Präparate enthaltend solche Verbindungen und die Verwendung von letzteren als pharmakologische Wirkstoffe.

In erster Linie betrifft die vorliegende Erfindung die Verbindungen der Formel

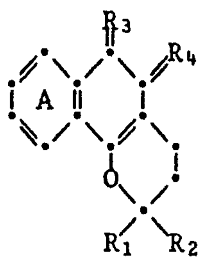

(I),

worin $R_1$ und $R_2$ unabhängig voneinander Wasserstoff, Niederalkyl oder Aryl oder zusammengenommen Niederalkylen bedeuten, einer der Reste $R_3$ und $R_4$ die Gruppe $=N-R$ (Ia) darstellt, worin R die Gruppe $-O-R_a$ (Ib) und $R_a$ Wasserstoff, Niederalkyl, Carboxyniederalkyl oder Niederalkoxycarbonylniederalkyl oder R die Gruppe $-N(R_b)-R_c$ (Ic) und $R_b$ und $R_c$ unabhängig voneinander Wasserstoff oder Niederalkyl oder einer der Reste $R_b$ und $R_c$ Wasserstoff und der andere Rest die Gruppe $-C(=X)-R_d$ darstellt, worin X Oxo und $R_d$ Niederalkoxy, Diniederalkylaminoniederalkyl, Triniederalkylammonioniederalkyl, Niederalkylenaminoniederalkyl, Oxaniederalkylenammonioniederalkyl, Thianiederalkylenaminoniederalkyl, gegebenenfalls durch Niederalkyl aza-substituiertes Azaniederalkylenaminoniederalkyl oder Pyridinioniederalkyl oder X Oxo oder Thioxo und $R_d$ Amino bedeuten, oder beide Reste

$R_b$ und $R_c$ zusammengenommen Niederalkylen, Oxaniederalkylen, Thianiederalkylen oder gegebenenfalls durch Niederalkyl aza-substituiertes Azaniederalkylen bedeuten, und der andere der beiden Reste $R_3$ und $R_4$ dieselben Bedeutungen hat oder Oxo bedeutet und der Ring A keine weiteren Substituenten hat oder zusätzlich durch Niederalkyl, Niederalkoxy und/oder Halogen substituiert ist, sowie Salze von solchen Verbindungen mit salzbildenen Eigenschaften.

Vorstehend wie nachfolgend mit "nieder" bezeichnete organische Reste und Verbindungen enthalten bis einschliesslich 7, vorzugsweise bis einschliesslich 4, und in erster Linie 1 oder 2 Kohlenstoffatome. Niederalkylenreste enthalten vorzugsweise 4 oder 5 Kohlenstoffatome.

Niederalkyl bedeutet in erster Linie Methyl und Aethyl, kann aber auch für n-Propyl, Isopropyl, n-Butyl, Isobutyl oder tert-Butyl, ferner für n-Pentyl, n-Hexyl und n-Heptyl stehen.

Aryl ist in erster Linie monocyclisches, carbocyclisches Aryl, z.B. gegebenenfalls substituiertes Phenyl, wobei Substituenten u.a. Niederalkyl, Niederalkoxy oder Halogen sein können.

Niederalkoxy ist insbesondere Methoxy oder Aethoxy, ferner n-Propoxy, Isopropoxy, n-Butoxy oder Isobutoxy sowie n-Pentyloxy.

Halogen bedeutet vorzugsweise Halogen mit einer Atomnummer von höchstens 35, d.h. Fluor, Chlor oder Brom.

Niederalkylen ist z.B. 1,4-Butylen, 1,5-Pentylen oder 1,6-Hexylen.

Carboxyniederalkyl ist in erster Linie Carboxymethyl und 2-Carboxyäthyl sowie 3-Carboxy-n-propyl.

Niederalkoxycarbonylniederalkyl ist in erster Linie Methoxy- oder Aethoxycarbonylmethyl und 2-Methoxy- oder 2-Aethoxycarbonyläthyl.

Diniederalkylaminoniederalkyl ist vorzugsweise Dimethylamino-, Diäthylamino-, Di-n-propylamino- oder N-Aethyl-N-methylaminomethyl und 2-Dimethylaminoäthyl.

Triniederalkylammonioniederalkyl ist vorzugsweise N-Diäthyl-N-methyl-ammonio- oder Trimethylammoniomethyl und 2-Trimethylammonioäthyl.

Niederalkylenaminoniederalkyl ist vorzugsweise 1-Pyrrolidinomethyl, 2-(1-Pyrrolidino)äthyl, 1-Piperidinomethyl, 2-(1-Piperidino)äthyl, 1-Hexahydroazepinomethyl und 2-(1-Hexahydroazepino)äthyl.

Oxaniederalkylenamino- und Thianiederalkylenaminoniederalkyl sind vorzugsweise 4-Morpholino- und 4-Thiomorpholinomethyl sowie 2-(4-Morpholino)- und 2-(4-Thiomorpholino)-äthyl.

Gegebenenfalls durch Niederalkyl aza-substituiertes Azaniederalkylenaminoniederalkyl ist vorzugsweise 1-Piperazinomethyl, 2-(1-Piperazino)äthyl, 4-Methyl- oder 4-Aethyl-1-piperazinomethyl sowie 4-Methyl- oder 4-Aethyl-2-(1-piperazino)äthyl.

Pyridinioniederalkyl ist vorzugsweise Pyridiniomethyl und 2-Pyridinioäthyl.

Oxaniederalkylen und Thianiederalkylen sind insbesondere 3-Oxa-1,5-pentylen bzw. 3-Thia-1,5-pentylen.

Gegebenenfalls durch Niederalkyl substituiertes Azaniederalkylen ist z.B. 3-Aza-1,5-pentylen oder 3-Methyl-3-aza-1,5-pentylen.

Vorzugsweise bedeutet in den Verbindungen der Formel I nur einer der Reste $R_3$ und $R_4$, in erster Linie der Rest $R_3$, die Gruppe der Formel Ia, während der andere Rest für Oxo steht.

Salze von Verbindungen der Formel I mit salzbildenden Eigenschaften sind in erster Linie pharmazeutisch verwendbare Salze z.B. Säureadditionssalze von solchen Verbindungen mit basischen Gruppen, wie Additionssalze mit anorganischen Säuren, z.B. Salzsäure, Schwefelsäure oder Phosphorsäure, oder organischen Säuren, wie Carbon- oder Sulfonsäuren, z.B. Essig-, Malein-, Fumar-, Aepfel-, Oxal-, Wein-, Zitronen-, Methansulfon- oder p-Toluolsulfonsäure. Quaternäre Stickstoffverbindungen, in denen der Rest $R_d$ für Triniederalkylammonio- oder Pyridinioniederalkyl steht, liegen vorzugsweise in der Form von pharmazeutisch verwendbaren quaternären Salzen vor, in denen z.B. das Anion von einer anorganischen Säure, wie einer Halogenwasserstoffsäure, z.B. Chlor-, Brom- oder Iodwasserstoffsäure, abgeleitet ist.

Es wird postuliert, dass die Verbindungen der vorliegenden Erfindung Prodrugs zur Hemmung der reversen Transkriptase, ein für Retroviren (oder Onconarviren), wie RNS-Tumor-und Leukämieviren, charakteristisches Enzym sind; Retroviren benötigen dieses Enzym für ihren natürlichen Replikationszyklus (Baltimore, Nature, Bd. 226, S. 1209 (1970); und Temin und Mizutani, Nature, Bd. 226, S. 1211 (1970)).

Die Hemmwirkung auf die reverse Transkriptase und die Virusreplikation kann anhand von in vivo-Testanordnungen festgestellt werden. So besitzen Verbindungen der Formel I in Tierexperimenten bei gewissen neoplastischen Krankheiten, die durch RNS-Tumorviren hervorgerufen werden, oder bei denen RNS-Tumorviren nachweisbar sind, eine starke Wirksamkeit. Sie verlängern z.B. die mittlere Ueberlebensdauer von Mäusen nach Infektion mit Rauscher Leukämie Virus (RLV, ein RNS-Tumorvirus).

Bei den Versuchen mit Rauscher Leukämie 4 Q (NIH) werden weibliche Balb/c-Mäuse (28 bis 34 Tage alt) mit 0,2 ml einer zehnfach verdünnten (Hanks Lösung) Virussuspension aus Milzmaterial von 4-6 Wochen vorher infizierten Mäusen infiziert. In einem Versuch werden je 15 Tiere entweder mit der Testverbindung oder als Kontrollen mit Placebo kontinuierlich per os behandelt. Die erste Verabreichung erfolgt 30 Minuten vor der Infektion, weitere Verabreichungen täglich einmal und fünfmal in der Woche während 4 Wochen. Als Kriterium für die Wirkung dient die Verlängerung der Ueberlebenszeit. Während bei den Kontrolltieren ab 28 Tagen nach der Infektion eine Leukämie mit leukämischen Blutzellen von über 50 000/$mm^3$ gefunden werden können und die Tiere zu sterben beginnen, wird die Ueberlebenszeit der mit 125 mg/kg p.o. der Testsubstanz behandelten weiblichen Mäuse signifikant verlängert. Sie beträgt z.B. für das 2,2-Dimethyl-3,4-dihydro-2H-naphtho[1,2-b]pyran-5,6-dion-6-semicarbazon bei 20-maliger p.o.-Verabreichung an weiblichen Balb/c-Mäusen von je 125 mg/kg (10 Tiere pro Gruppe) 53,5 Tage, verglichen mit der Ueberlebenszeit der Kontrolltiere von 26.0 Tagen ($p \leq 0,01$ Cox-Test).

Die pharmakologische Wirksamkeit der Verbindungen der vorliegenden Erfindung ist begleitet von einer guten Verträglichkeit. So liegt die maximal tolerierte Dosis für das oben genannte 2,2-Dimethyl-3,4-dihydro-2H-naphtho[1,2-b]pyran-5,6-dion-6-semicarbazon in Mäusen nach einmaliger p.o- und i.p.-Verabreichung und anschliessender 7-tägiger Beobachtungsdauer über 2500 mg/kg.

Der Nachweis von Erkrankungen, die durch Retroviren verursacht werden, basiert z.B. auf den folgenden Befunden: Der Nachweis von Typ C-Retroviren und deren Bildung von reverser Transkriptase beim Menschen erfolgte zuerst bei einer T-Zellen-Leukämie; das Virus wurde Human T-cell leukaemia virus (HTLV-I) genannt. Bei weiteren T-Zellen-Leukämien und -Lymphomen wurden die gleichen Viren und ein ähnliches Retrovirus (HTLV-II) gefunden (Gallo, Cancer Surveys (Ed. Franks, Wyke und Weiss), Bd. 3, S. 113-160 (Oxford Univ. Press, 1984)). Schliesslich wurde ein derartiges Virus auch im Zusammenhang mit AIDS (Acquired Immune Deficiency Syndrome)-Erkrankungen isoliert (Gottlieb et al., Morbid. Mortal. Weekly Rep., Bd. 30, S. 25,(1981); Friedman-Kien et al., Morbid. Mortal. Weekly Rep., Bd. 30, S. 305 (1981); Gottlieb et al., New Engl. J. Med., Bd. 305, S. 1425 (1981); Masur, New Engl. J. Med., Bd. 305, S. 1431 (1981); Siegal et al.,

New Engl. J. Med., Bd. 305, S. 1439 (1981); CDC Task Force on Kaspoi's Sarcoma and Opportunistic Infections, New Engl. J. Med., Bd. 306, S. 248 (1982)); dieses wurde mit HTLV-III bezeichnet (Essex et al., Science, Bd. 220, S. 859 (1983), Gelmann et al., Bd. 220, S. 862 (1983); Gallo et al., Science, Bd. 220, S. 865 (1983); Gallo, Cancer Surveys, Bd. 3 S. 113 (1984); Barré-Sinoussi et al., Science Bd. 220, 868 (1983); Hirsch und Levy, Viruses in Human Malignancy and AIDS, ASCO/AACR Symposium, May 1984, Toronto).

Diese der HTLV-Familie angehörenden Retroviren benötigen die reverse Transkriptase für die Bildung einer Doppelstrang-DNA (Provirus), welche in das Zell-Genom "integriert" wird und zur malignen Transformation führen kann (Strayer und Gillespie, The Nature and Organization of Retroviral Genes in Animal Cells, Virology Monographs, Bd. 17 (Springer Ed., 1980)).

Während nach der Induktion maligner leukämischer, lymphatischer oder tumoröser Neubildungen durch Retroviren eine Abnahme der reversen Transkriptase und des Virus HTLV-I und -II nachgewiesen werden kann und sekundäre, durch diese Viren induzierte Oncogene die Replikation der malignen Zellen steuern, ist das Virus HTLV-III und die reverse Transkriptase bei AIDS nicht nur im Frühstadium, sondern während des ganzen Krankheitsverlaufs nachweisbar. Die laufende Infektion und Funktionsstörung von immunkompetenten T-Helfer Zellen führt schliesslich zum Zusammenbruch des Immunsystems mit letalem Ausgang. Es ist anzunehmen, dass eine Hemmung der reversen Transkriptase und der Virusreplikation bei positivem Enzym- und Virusantigen-Nachweis (Beardsley, Nature, Bd. 311, S. 195 (1984) in Risikopatienten, z.B. Homosexuellen, den Krankheitsprozess beeinflusst. Bei der Induktion von Leukämien und Lymphomen durch Retroviren (HTLV-I und HTLV-II), wie möglicherweise auch bei durch Retroviren induzierten Sarkomen oder Mammakarzinomen, sollte dagegen eine prophylaktische Anwendung möglich sein, sofern eine leicht und breit verfügbare diagnostische Erfassung von derartigen Risikopatienten vorausgesetzt werden kann.

Auch im Zusammenhang mit der nicht-A- und nicht-B-Hepatitis ist die reverse Transkriptase als spezifisches Enzym in Assoziation mit Viruspartikeln gefunden worden (Seto et al., Lancet, S. 941 (1984)).

Die Verbindungen der vorliegenden Anmeldung können deshalb zur Prophylaxe und Therapie von Krankheiten, bei denen die reverse Transkriptase von Bedeutung ist, vor allem von durch Typ-C-Retroviren verursachten oder mitverursachten malignen Erkrankungen, aber auch von gewissen Immunkrankheiten und Autoimmunkrankheiten Verwendung finden. Als Tumorkrankheiten kommen in erster Linie durch Retroviren verursachte Leukämien, Lymphome und Lymphosarkome, sowie Osteosarkome und Mammakarzinome in Betracht. Die Verbindungen der Erfindung eignen sich besonders auch zur Rezidivprophylaxe nach chirurgischer Therapie, Strahlentherapie oder zytostatischer oder antimetabolischer Chemotherapie. Als Immunkrankheit sei AIDS, als Autoimmunkrankheit systemischer Lupus erythematosus genannt, bei denen nach neueren Befunden RNS-Tumorviren auch eine wichtige Rolle zu spielen scheinen (Dennman, Med., Biol., Bd. 53, S 61 (1975); Panem et al., New England J. Med., Bd. 295, 470 (1976)).

Die Erfindung betrifft insbesondere Verbindungen der Formel I, worin $R_1$ Wasserstoff oder Niederalkyl, $R_2$ Wasserstoff, Niederalkyl oder Phenyl bedeutet, einer der Reste $R_3$ und $R_4$, vorzugsweise $R_3$, die Gruppe =N-R (Ia) darstellt, worin R die Gruppe $-O-R_a$ (Ib) und $R_a$ Wasserstoff, Niederalkyl, Carboxyniederalkyl oder Niederalkoxycarbonylniederalkyl oder R die Gruppe $-N(R_b)-R_c$ (Ic) und $R_b$ und $R_c$ unabhängig voneinander Wasserstoff oder Niederalkyl oder einer der Reste $R_b$ und $R_c$ Wasserstoff und der andere Rest die Gruppe $-C(=X)-R_d$ darstellt, worin X Oxo und $R_d$ Niederalkoxy, Diniederalkylaminoniederalkyl, Triniederalkylammonioniederalkyl, Niederalkylenaminoniederalkyl, 4-Morpholinoniederalkyl, 4-Niederalkyl-1-piperazinoniederalkyl oder Pyridinioniederalkyl oder X Oxo oder Thioxo und $R_d$ Amino bedeuten, oder beide Reste $R_b$ und $R_c$ zusammengenommen Niederalkylen, 3-Oxa-1,5-pentylen oder 3-Niederalkyl-3-aza-1,5-pentylen bedeuten, und der andere der beiden Reste $R_3$ und $R_4$, vorzugsweise $R_4$, für Oxo steht, und der Ring A keine weiteren Substituenten hat oder zusätzlich durch Niederalkyl, Niederalkoxy oder Halogen substituiert ist, wobei mit "nieder" bezeichnete Reste vorzugsweise 1 oder 2 Kohlenstoffatome, Niederalkylenreste 4 oder 5 Kohlenstoffatome enthalten, und Halogen eine Atomnummer von höchstens 35 hat, und Salze, insbesondere pharmazeutisch verwendbare Salze, von solchen Verbindungen mit salzbildenden Eigenschaften.

Die Erfindung betrifft in erster Linie Verbindungen der Formel I, worin $R_1$ für Wasserstoff oder Niederalkyl und $R_2$ für Niederalkyl oder Phenyl stehen, $R_3$ eine Gruppe der Formel =N-R (Ia) darstellt, worin R die Gruppe $-O-R_a$ (Ib) und $R_a$ Niederalkyl, Carboxyniederalkyl oder Niederalkoxycarbonylniederalkyl oder R die Gruppe $-N(R_b)-R_c$ (Ic) eine der Reste $R_b$ und $R_c$ Wasserstoff und der andere Rest die Gruppe $-C(=X)-R_d$ darstellt, worin X Oxo oder Thioxo und $R_d$ Amino bedeuten oder beide Reste $R_b$ und $R_c$ zusammengenommen Niederalkylen, 3-Oxa-1,5-pentylen oder 3-Niederalkyl-3-aza-1,5-pentylen bedeuten, und $R_4$ für Oxo steht, und der Ring A keine weiteren Substituenten hat, wobei mit "nieder" bezeichnete Reste vorzugsweise 1 oder 2 Kohlenstoffatome, Niederalkylenreste 4 oder 5 Kohlenstoffatome enthalten, und Salze, insbesondere pharmazeutisch verwendbare Salze, von solchen Verbindungen mit salzbildenden Eigenschaften.

Die Erfindung betrifft vor allem Verbindungen der Formel I, worin $R_1$ für Wasserstoff oder Niederalkyl, z.B. Methyl, und $R_2$ für Niederalkyl, z.B. Methyl, oder Phenyl stehen, $R_3$ die Gruppe =N-R (Ia) und R die Gruppe $-N(R_b)-R_c$ (Ic) darstellt, wobei einer der Reste $R_b$ und $R_c$ Wasserstoff und der andere Rest die Gruppe $-C(=X)-R_d$ (Id) bedeutet, worin X für Oxo und $R_d$ für Amino stehen, wobei mit "nieder" bezeichnete Reste in erster Linie ein, ferner auch zwei Kohlenstoffatome enthalten.

Die Verbindungen der vorliegenden Erfindung werden nach an sich bekannten Methoden hergestellt, z.B. indem man eine Verbindung der Formel

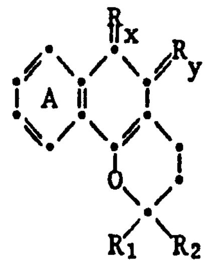 (II),

worin eine der Gruppen $R_x$ und $R_y$ für Oxo steht und die andere Oxo oder die Gruppe =N-R (Ia) darstellt, und $R_1$ und $R_2$ sowie der Ring A in der Formel II und R in der Gruppe Ia die oben gegebenen Bedeutungen haben, mit einer Verbindung der Formel $H_2N-R$ (III), worin R die oben gegebene Bedeutung hat, umsetzt und, wenn erwünscht, eine erhaltene Verbindung in eine andere erfindungsgemässe Verbindung überführt und/oder, wenn erwünscht, ein erhaltenes Salz in die freie Verbindung oder in ein anderes Salz überführt oder eine erhaltene freie Verbindung mit salzbildenden Gruppen in ein Salz überführt.

Als Ausgangsstoffe werden vorzugsweise Verbindungen der Formel II verwendet, in denen beide Reste $R_x$ und $R_y$ für Oxo stehen. Die Verbindungen der Formel III können in freier Form oder in Form von Säureadditionssalzen, wie Salzen mit anorganischen Säuren, z.B. Salz- oder Schwefelsäure, oder organischen Säuren eingesetzt werden.

Die Reaktion wird in an sich bekannter Weise durchgeführt, vorzugsweise in Gegenwart einer Base, wie eines Alkalimetallniederalkanoats, z.B. Natriumacetat, und/oder eines, vorzugsweise polaren, Verdünnungsmittels, wie eines Niederalkanols, z.B. Aethanol, eines zyklischen Aethers, z.B. Tetrahydrofuran oder Dioxan, oder eines Amids, z.B. Dimethylformamid, unter Kühlen, bei Raumtemperatur oder vorzugsweise unter Erwärmen, z.B. in einem Temperaturintervall von etwa 10°C und etwa 120°C, vorzugsweise 50°C und etwa 100°C, falls notwendig, in einem geschlossenen Gefäss unter Druck und/oder in einer Inertgasatmosphäre.

Erhaltene Verbindungen können in andere Verbindungen der Erfindung übergeführt werden, so z.B. solche der Formel I mit einer Gruppe der Formel $-C(=X)-R_d$ (Id), worin $R_d$ für eine Diniederalkylaminoniederalkylgruppe steht, durch Behandeln mit einem reaktionsfähigen Ester eines Niederalkanols, wie einem Niederalkylhalogenid, z.B. -chlorid, -bromid oder -iodid, in Verbindungen mit einer Triniederalkylammonioniederalkylgruppe $R_d$.

Verfahrensgemäss erhältliche Salze von Verbindungen der Formel I können in an sich bekannter Weise, z.B. Säureadditionssalze durch Behandeln mit einer geeigneten Base, in die freien Verbindungen übergeführt werden. Quaternäre Salze können z.B. durch Behandeln mit einer geeigneten Säure oder einem Anionenaustauscher in ein anderes quaternäres Salz übergeführt werden. Verbindungen der Formel I mit salzbildenden Eigenschaften können z.B. durch Behandeln mit einer Säure oder einem geeigneten Ionenaustauscher in die gewünschten Salze übergeführt werden.

Die im obigen Verfahren verwendeten Ausgangsstoffe der Formeln II und III sind bekannt oder können, falls sie neu sind, in an sich bekannter Weise hergestellt werden.

Die vorliegende Erfindung betrifft ebenfalls pharmazeutische Präparate, die als Wirkstoffe eine der erfindungsgemässen Verbindungen enthalten. Bei den erfindungsgemässen pharmazeutischen Präparaten handelt es sich um solche zur enteralen, wie oralen oder rektalen oder parenteralen, z.B. i.v., i.m. oder rektalen, Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Spezies, deren Alter und individuellem Zustand, sowie von der Applikationsweise ab.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Erfindungsgemässe pharmazeutische Präparate in Dosiseinheitsform, wie Dragées, Tabletten, Kapseln, Suppositorien oder Ampullen, enthalten von etwa 0,05 g bis etwa 1,5 g, vorzugsweise von etwa 0,1 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls nach Zugabe von zusätzlichen Hilfsstoffen, zu Tabletten oder Dragées-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglycol.

Dragées-Kerne werden mit geeigneten, gegebenenfalls Magensaftresistenten, Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragées-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffine, Polyäthylenglycole oder höhere Alkanole. Ferner können auch Gelatine-Rektalkapseln verwendet werden, die aus einer Kombination des Wirkstoffes mit einer Grundmasse bestehen; als Grundmassenstoffe kommen z.B. flüssige Triglyceride, Polyäthylenglycole oder Paraffine in Frage.

Zur parenteralen Verabreichung kommen z.B. Suspensionen von Verbindungen der vorliegenden Erfindung, wie entsprechende ölige Injektionssuspensionen, in Betracht, wobei man geeignete lipophile Lösungsmittel oder Vehikel, wie fette Oele, z.B. Sesamöl, oder synthetische Fettsäureester, z.B. Aethyloleat oder Triglyceride, verwendet, oder es eignen sich wässrige Injektionssuspensionen, welche viskositätserhöhende Stoffe, z.B. Natriumcarboxymethylcellulose, Sorbit und/oder Dextran und gegebenenfalls auch Stabilisatoren enthalten.

Zur parenteralen Verabreichung eignen sich insbesondere Liposomendispersionen mit Verbindungen der Formel I. Die Liposomendispersionen werden aus mindestens zwei Lipidkomponenten, z.B. Phosphatidylserin und Phosphatidyläthanolamin oder Phosphatidylserin und Phosphatidylcholin, gebildet, welche Verbindungen (I) verkapseln.

Zur Herstellung der Liposomendispersion eignet sich für die eine Lipidkomponente Phosphatidylserin natürlicher Herkunft mit verschiedenen oder identischen $C_{10}$-$C_{20}$-Alkanoylresten, z.B. n-Dodecanoyl, n-Tetradecanoyl, n-Hexadecanoyl oder n-Octadecanoyl, oder $C_{10}$-$C_{20}$-Alkenoylresten, z.B. 9-cis-Dodecenoyl, 9-cis-Tetradecenoyl, 9-cis-Hexadecenoyl, 6-cis-, 6-trans-, 9-cis-, 9-trans- oder 11-cis-Octadecenoyl oder 9-cis-Icosenoyl, z.B. Phosphatidylserin aus dem Rinderhirn, oder synthetisches Phosphatidylserin mit identischen $C_{10}$-$C_{20}$-Alkenoylresten, z.B. Natrium-1,2-di-(9-cis-octadecenoyl)-3-sn-phosphatidyl-(S)-serin, und für die andere Lipidkomponente Phosphatidylcholin natürlicher Herkunft mit verschiedenen oder identischen $C_{10}$-$C_{20}$-Alkanoylresten, z.B. Phosphatidylcholin aus dem Hühnerei oder aus Sojaöl, synthetisches Phosphatidylcholin mit identischen $C_{10}$-$C_{20}$-Alkanoylresten, z.B. Dimyristoyl-, Distearoyl- oder Dipalmitoylphosphatidylcholin, oder synthetisches Phosphatidylcholin mit einem $C_{10}$-$C_{20}$-Alkanoyl- und einem $C_{10}$-$C_{20}$-Alkenoylrest, z.B. 1-n-Hexadecanoyl-2-(9-cis-octadecenoyl)-3-sn-phosphatidylcholin.

Die Liposomendispersion lässt sich herstellen, indem man beispielsweise ein Lyophilisat aus den Lipidkomponenten und der Verbindung (I) gemäss dem in der Deutschen Offenlegungsschrift 28 18 655 beschriebenen Verfahren hergestellt, vorzugsweise nach Lösen der Lipidkomponenten und des Wirkstoffs in tert-Butanol, und Abziehen des Lösungsmittels bei Temperaturen unterhalb -20°, und dieses Lyophilisat in einer isotonischen Pufferlösung dispergiert. Die Dispersion erfolgt durch Schütteln (Vortex-Mischer) oder Rühren der wässrigen Phase. Die Liposomendispersion kann anschliessend durch Zentrifugation angereichert und/oder durch Gelfiltration oder Extrusion durch geradporige Filter abgetrennt werden.

Dieses Verfahren eignet sich, wenn lipophile bzw. in organischen Lösungsmitteln, z.B. tert-Butanol, lösliche Verbindungen (I) verwendet werden. Bei wasserlöslichen Verbindungen (I) stellt man ein Lyophilisat nur aus den Lipidkomponenten her und dispergiert dieses in wässriger Phase, welche die gelöste Verbindung (I) enthält.

Die Erfindung betrifft ebenfalls ein Verfahren zur Behandlung von Krankheiten, bei denen, wie oben beschrieben worden ist, die reverse Transkriptase von Bedeutung ist, dadurch gekennzeichnet, dass man eine prophylaktisch oder therapeutisch wirksame Menge einer erfindungsgemässen Verbindung verabreicht. Dabei verwendet man in erster Linie die oben genannten pharmazeutischen Präparate, wobei man einem Warmblüter von etwa 70 kg Gewicht eine tägliche Dosis von etwa 0,1 g bis etwa 5 g, vorzugsweise von etwa 0,5 g bis etwa 1,5 g einer Verbindung der vorliegenden Erfindung verabreicht.

Die nachfolgenden Beispiele illustrieren die vorliegende Erfindung; Temperaturen werden in Celsiusgraden angegeben.

Beispiel 1: 2,2-Dimethyl-3,4-dihydro-2H-naphtho[1,2-b]pyran-5,6-dion-6-semicarbazon

Eine Lösung von 6.0 g 2,2-Dimethyl-3,4-dihydro-2H-naphtho[1,2-b]-pyran-5,6-dion (ß-Lapachon) in 200 ml eines 1:1-Gemisches von Aethanol und Wasser wird durch Erwärmen auf 70° hergestellt und die Lösung mit 2,03 g Natriumacetat und 2,76 g Semicarbazid-hydrochlorid versetzt. Die sich bildende gelbe Suspension wird während 3 Stunden bei 80° gerührt, auf 10° abgekühlt und filtriert. Der Filterrückstand wird mit 100 ml Wasser gewaschen und in 1000 ml Aceton, worin dieser nur zum Teil löslich ist, aufgekocht und das Gemisch filtriert. Die aus dem Filtrat erhältliche Titelverbindung schmilzt bei 249-253° (mit Zersetzen). Eine weitere Menge der erwünschten Verbindung ist in der angegebenen Menge Aceton nicht löslich und im Filterrückstand enthalten. Weiteres Material kann aus der Mutterlauge gewonnen werden.

Beispiel 2: 2,2-Dimethyl-3,4-dihydro-2H-naphtho[1,2-b]pyran-5,6-dion-6-thiosemicarbazon

Ein Gemisch von 15 g 2,2-Dimethyl-3,4-dihydro-2H-naphtho[1,2-b]-pyran-5,6-dion(ß-Lapachon), 15,8 g Thiosemicarbazid und 15,8 g Natriumacetat in 500 ml eines 1:1-Gemisches von Aethanol und Wasser wird während 4 Stunden unter Rückfluss erhitzt und dann auf Raumtemperatur abgekühlt. Der rötliche Niederschlag wird abfiltriert, der Rückstand mit Wasser gewaschen, während 16 Stunden bei 70° unter vermindertem Druck getrocknet und dann aus 3000 ml Aceton unter Einengen auf das halbe Volumen umkristallisiert und mit Diäthyläther gewaschen. Die so erhältliche Titelverbindung schmilzt bei 249-250°. Eine weitere Menge des gewünschten Produkts kann aus der Mutterlauge isoliert werden.

Beispiel 3: 2,2-Dimethyl-3,4-dihydro-2H-naphtho[1,2-b]pyran-5,6-dion-6-(2-dimethylaminoacetylhydrazon)-hydrochlorid

Eine Suspension von 7,2 g 2,2-Dimethyl-3,4-dihydro-2H-naphtho-[1,2-b]pyran-5,6-dion(ß-Lapachon) und 6,1 g N,N-Dimethylglycinhydrazid-hydrochlorid in 70 ml Essigsäure wird während 10 Minuten bei einer Badtemperatur von 100° erwärmt. Das Lösungsmittel wird

unter vermindertem Druck entfernt, und der Rückstand in heissem Dimethylformamid gelöst. Beim Erkalten bildet sich ein organgeroter, kristalliner Niederschlag, der bei 90° unter Hochvakumm getrocknet wird. Man erhält so die Titelverbindung, welche bei 185° (mit Zersetzen) schmilzt.

Beispiel 4: 2,2-Dimethyl-3,4-dihydro-2H-naphtho[1,2-b]pyran-5,6-dion-6-(2-trimethylammonioacetylhydrazon)-chlorid

Ein Gemisch von 7,2 g 2,2-Dimethyl-3,4-dihydro-2H-naphtho[1,2-b]-pyran-5,6-dion(β-Lapachon) und 6,72 g Trimethylammonioacetylhydrazid-chlorid in 50 ml Essigsäure wird während 10 Minuten bei 100° erwärmt und dann unter vermindertem Druck zur Trockne eingedampft. Das erhaltene, ölige Produkt wird durch mehrmaliges Behandeln mit 1,2-Dimethoxyäthan kristallisiert. Das kristalline Material wird abfiltriert, in Aethanol gelöst und die Lösung mit der gleichen Menge Essigsäureäthylester verdünnt. Beim Kühlen auf 0° bildet sich die Titelverbindung in Form eines organge-farbenen, kristallinen Niederschlags, der bei 210° (mit Zersetzung) schmilzt.

Beispiel 5: 2,2-Dimethyl-3,4-dihydro-2H-naphtho[1,2-b]pyran-5,6-dion-6-(2-pyridinioacetylhydrazon)-chlorid

Eine Suspension von 7,2 g 2,2-Dimethyl-3,4-dihydro-2H-naphtho-[1,2-b]pyran-5,6-dion(β-Lapachon) und 7,5 g 1-(2-Hydrazino-2-oxoäthyl)-pyridiniochlorid in 50 ml Essigsäure wird während 10 Minuten bei 100° erwärmt und dann unter vermindertem Druck zur Trockne eingedampft. Das erhaltene ölige Produkt wird durch mehrmaliges Behandeln mit 1,2-Dimethoxyäthan kristallisiert. Das kristalline Material wird abfiltriert und aus Isopropanol und anschliessend aus Acetonitril kristallisiert. Die so hergestellte Titelverbindung wird als Sesquihydrat erhalten und zersetzt sich bei 225°.

Beispiel 6: 2,2-Dimethyl-3,4-dihydro-2H-naphtho[1,2-b]pyran-5,6-dion-6-oxim

Eine Lösung von 2,0 g 2,2-Dimethyl-3,4-dihydro-2H-naphtho[1,2-b]-pyran-5,6-dion (β-Lapachon) in 125 ml Aethanol und 4,2 ml Triäthylamin wird mit 1,73 g Hydroxylamin-hydrochlorid versetzt. Die

Reaktionslösung wird während 6 Stunden bei 25°C gerührt und auf 5° abgekühlt. Die Kristalle werden abgenutscht, mit Wasser gewaschen und getrocknet. Die erhaltene Titelverbindung hat einen Schmelzpunkt von 169-171°.

Beispiel 7: 2,2-Dimethyl-3,4-dihydro-2H-naphtho[1,2-b]pyran-5,6-dion-6-methoxim

Eine Lösung von 2,49 g 2,2-Dimethyl-3,4-dihydro-2H-naphtho[1,2-b]-pyran-5,6-dion (β-Lapachon) in 155 ml Aethanol und 21 ml Wasser wird mit 4,22 g Natriumacetat und 4,3 g Methylhydroxylamin-hydrochlorid versetzt. Die Reaktionslösung wird während 4 Stunden unter Rückfluss erhitzt und auf 5° abgekühlt. Die Kristalle werden abgenutscht, mit Wasser gewaschen, getrocknet und aus Heptan umkristallisiert. Die erhaltene Titelverbindung hat einen Schmelzpunkt von 79-80°.

Beispiel 8: 2,2-Dimethyl-3,4-dihydro-2H-naphtho[1,2-b]pyran-5,6-chinon-6-äthoxim

Eine Lösung von 2,49 g 2,2-Dimethyl-3,4-dihydro-2H-naphtho[1,2-b]-pyran-5,6-dion (β-Lapachon) in 150 ml Aethanol und 20 ml Wasser wird mit 4,22 g Natriumacetat und 5,0 g Aethylhydroxylaminhydrochlorid versetzt. Die Reaktionslösung wird während 4 Stunden unter Rückfluss erhitzt und dann zur Trockne eingedampft. Der Rückstand wird in Methylenchlorid gelöst und die organische Phase zweimal mit Wasser gewaschen. Die organische Phase wird getrocknet, zur Trockne eingedampft und der Rückstand aus Hexan kristallisiert. Die erhaltene Titelverbindung schmilzt bei 72-73°.

Beispiel 9: 2,2-Dimethyl-3,4-dihydro-2H-naphtho[1,2-b]pyran-5,6-dion-6-äthoxycarbonylmethyloxim

Eine Lösung von 2,0 g 2,2-Dimethyl-3,4-dihydro-2H-naphtho[1,2-b]-pyran-5,6-dion (β-Lapachon) in 1000 ml Aethanol wird mit 10 g O-Carboxymethylhydroxylaminhemihydrochlorid versetzt und während 2 Stunden bei Raumtemperatur gerührt, wobei sich die Lösung von orange nach gelb färbt. Die Lösung wird zur Trockne eingedampft, der Rückstand in Methylenchlorid gelöst und zweimal mit Natriumbicarbonatlösung gewaschen. Die organische Phase wird dann zweimal mit

verd. HCl und einmal mit Wasser gewaschen, getrocknet und eingedampft. Aus dem Rückstand kristallisiert aus Aether/Petroläther die Titelverbindung in gelben Nadeln. Schmelzpunkt 93-95°.

Beispiel 10: 2,2-Dimethyl-3,4-dihydro-2H-naphtho[1,2-b]pyran-5,6-dion-6-carboxymethyloxim

Eine Lösung von 1,56 g 2,2-Dimethyl-3,4-dihydro-2H-naphtho[1,2-b]-pyran-5,6-dion (β-Lapachon) in 30 ml THF wird mit 0,78 g O-Carboxymethylhydroxylaminhemihydrochlorid, gelöst in 8 ml THF-$H_2O$ (1:1), versetzt und 8 Stunden bei Raumtemperatur gerührt. Die Lösung wird zur Trockne eingedampft, der Rückstand in Methylenchlorid gelöst und zweimal mit verd. HCl und einmal mit $H_2O$ gewaschen. Die organische Phase wird getrocknet und eingedampft. Der Rückstand wird in 120 ml warmem Aether aufgeschlämmt und unter Rückfluss erwärmt, wobei die Titelverbindung als gelbe Kristalle mit einem Schmelzpunkt von 165-167° erhalten wird.

Beispiel 11: 2,2-Dimethyl-3,4-dihydro-2H-naphtho[1,2-b]pyran-5,6-dion-6-carboxymethyloxim-natriumsalz

1 g 2,2-Dimethyl-3,4-dihydro-2H-naphtho[1,2-b]pyran-5,6-dion-6-(carboxymethyl)-oxim wird in ca. 20 ml Dioxan gelöst und mit äquimolarer Menge 1N Natronlauge versetzt. Die klare Lösung wird gefriergetrocknet, wobei die Titelverbindung als gelbes Pulver erhalten wird.

Beispiel 12: 2,2-Dimethyl-3,4-dihydro-2H-naphtho[1,2-b]pyran-5,6-dion-6-äthoxycarbonylhydrazon

Eine Lösung von 2,0 g 2,2-Dimethyl-3,4-dihydro-2H-naphtho[1,2-b]-pyran-5,6-dion (β-Lapachon) in 100 ml Aethanol und 20 ml Wasser wird mit 0,75 g Natriumacetat und 4,8 g Hydrazincarbonsäureäthylester versetzt und 50 Stunden unter Rückfluss erhitzt. Die Reaktionsmischung wird eingeengt, der Rückstand in Methylenchlorid gelöst und zweimal mit Wasser gewaschen. Die organische Phase wird getrocknet, eingedampft und der Rückstand an 100 g Silicagel chromatographiert. Es wird die Titelverbindung in Form gelber Kristalle mit einem Schmelzpunkt von 90-95° erhalten.

Beispiel 13: Tabletten, enthaltend 500 mg 2,2-Dimethyl-3,4-dihydro-2H-naphtho[1,2-b]pyran-5,6-dion-6-semicarbazon, können wie folgt hergestellt werden:

Zusammensetzung (für 10 000 Tabletten):

| | |
|---|---|
| 2,2-Dimethyl-3,4-dihydro-2H-naphtho[1,2-b]-pyran-5,6-dion-6-semicarbazon | 5000,00 g |
| Weizenstärke | 790,00 g |
| Stearinsäure | 30,00 g |
| Magnesiumstearat | 30,00 g |
| Talk | 400,00 g |

Der Wirkstoff wird mit verkleisterter Weizenstärke, die durch Anrühren von 500 g Weizenstärke mit etwa 1300 ml entmineralisiertem Wasser erhalten wird, und mit weiteren 600 ml entmineralisiertem Wasser gleichmässig befeuchtet, zu einer schwach plastischen Masse geknetet und durch ein Sieb mit einer Maschenweite von etwa 3 mm getrieben. Das Granulat wird anschliessend getrocknet und erneut durch ein Sieb geschlagen. Dem trockenen und auf eine einheitliche Korngrösse gebrachten Granulat werden das Magnesiumstearat, die Stearinsäure, der Talk und der Rest der Weizenstärke zugemischt, und das erhaltene Gemisch wird zu Tabletten verpresst.

Beispiel 14: Eine 10 %ige Suspension enthaltend 2,2-Dimethyl-3,4-dihydro-2H-naphtho[1,2-b]pyran-5,6-dion-6-semicarbazon kann wie folgt hergestellt werden:

Zusammensetzung (für 5000 ml):

| | |
|---|---|
| 2,2-Dimethyl-3,4-dihydro-2H-naphtho[1,2-b] pyran-5,6-dion-6-semicarbazon | 500,00 g |
| Propylenglycol | 500,00 g |
| Hydroxypropylmethylcellulose | 25,00 g |
| Zitronensäure | 5,00 g |
| Saccharin-natrium | 2,50 g |
| 4-Hydroxybenzoesäuremethylester | 6,00 g |

| | |
|---|---|
| 4-Hydroxybenzoesäurepropylester | 1,50 g |
| Himbeeraroma | 5,00 g |
| 70 %ige wässrige Sorbitlösung | 1250 ml |
| entmineralisiertes Wasser | q.s. |

Eine Suspension des Wirkstoffs in 250 g Propylenglycol und 1250 ml entmineralisiertem Wasser wird in einer Kolloidmühle gemahlen. Die feine Suspension, mit einer durchschnittlichen Teilchengrösse des Wirkstoffs von unter 10 µm, wird in einer Lösung der Hydroxypropylmethylcellulose, der Zitronensäure und des Saccharin-natriums in 1250 ml entmineralisiertem Wasser und in der Sorbit-Lösung dispergiert. Unter Rühren werden zu dieser Suspension eine Lösung des 4-Hydroxybenzoesäuremethylesters und des 4-Hydroxybenzoesäurepropylesters in 250 g Propylenglycol und das Himbeeraroma gegeben. Nach dem Ergänzen mit entmineralisiertem Wasser auf das Volumen von 5000 ml erhält man eine Suspension, die 500 mg des Wirkstoffs in 5 ml enthält.

Beispiel 15: Kapseln, enthaltend je 300 mg 2,2-Dimethyl-3,4-dihydro-2H-naphtho[1,2-b]pyran-5,6-dion-6-semicarbazon, können wie folgt hergestellt werden:

Zusammensetzung (für 10 000 Kapseln)

| | |
|---|---|
| 2,2-Dimethyl-3,4-dihydro-2H-naphtho[1,2-b]-pyran-5,6-dion-6-semicarbazon | 3000,00 g |
| Magnesiumstearat | 100,00 g |

Der Wirkstofff wird mit dem Magnesiumstearat vermischt und je 0,31 g des Gemisches mit Hilfe einer Verkapselungsmaschine in Hartgelatinekapseln abgefüllt.

Beispiel 16: Eine wässrige Injektionssuspension (für intramuskuläre Verabreichung), enthaltend 1,0 g 2,2-Dimethyl-3,4-dihydro-2H-naphtho-1,2-b]pyran-5,6-dion-6-semicarbazon pro 5 ml, kann wie folgt hergestellt werden:

Suspensionsmedium:

| | | |
|---|---|---|
| Natriumsalz von Carboxymethylcellulose | | 0,10 % |
| Gemisch von Natriumhydrogenphosphat und Dinatriumhydrogenphosphat | | 0,15 % |
| Natriumchlorid | | 0,75 % |
| Natriumsalz der S-Aethylmercuri-thiosalicylsäure (Thiomersal) | | 0,02 % |
| 1,2 Propylenglycol | | 20,00 % |
| Destilliertes Wasser | ad | 100,00 % |

Der sterile 2,2-Dimethyl-3,4-dihydro-2H-naphtho[1,2-b]pyran-5,6-dion-6-semicarbazon-Wirkstoff wird unter antimikrobiellen Bedingungen mit dem Suspensionsmedium so verarbeitet, dass eine sterile Suspension erhalten wird, die 1,0 g des Wirkstoffs in 5 ml erhält.

Beispiel 17: Eine wässrige Injektionssuspension (für intramuskuläre Verabreichung), enthaltend 0,75 g 2,2-Dimethyl-3,4-dihydro-2H-naphtho[1,2-b]pyran-5,6-dion-6-semicarbazon pro 5 ml, kann wie folgt hergestellt werden:

Suspensionsmedium:

| | |
|---|---|
| Natriumchlorid | 0,90 % |
| Natriumsalz der S-Aethylmercuri-thiosalicylsäure (Thiomersal) | 0,02 % |
| Polyoxyäthylen(20)Sorbitan-Monostearat (Molekulargewicht: 1311,7 g; Tween 60®) | 0,75 % |
| Natriumsalz von Carboxymethylcellulose | 0,50 % |

Der sterile Wirkstoff wird unter antimikrobiellen Bedingungen mit dem Suspensionsmedium so verarbeitet, dass man eine sterile Suspension erhält, die 0,75 g Wirkstoff in 5 ml enthält.

Ansprüche (für alle benannten Vertragsstaaten ausser Oesterreich)

1. Verbindungen der Formel

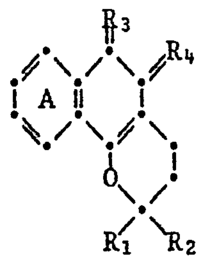

(I),

worin $R_1$ und $R_2$ unabhängig voneinander Wasserstoff, Niederalkyl oder Aryl oder zusammengenommen Niederalkylen bedeuten, einer der Reste $R_3$ und $R_4$ die Gruppe =N-R (Ia) darstellt, worin R die Gruppe $-O-R_a$ (Ib) und $R_a$ Wasserstoff, Niederalkyl, Carboxyniederalkyl oder Niederalkoxycarbonylniederalkyl oder R die Gruppe $-N(R_b)-R_c$ (Ic) und $R_b$ und $R_c$ unabhängig voneinander Wasserstoff oder Niederalkyl oder einer der Reste $R_b$ und $R_c$ Wasserstoff und der andere Rest die Gruppe $-C(=X)-R_d$ darstellt, worin X Oxo und $R_d$ Niederalkoxy, Diniederalkylaminoniederalkyl, Triniederalkylammonioniederalkyl, Niederalkylenaminoniederalkyl, Oxaniederalkylenammonioniederalkyl, Thianiederalkylenaminoniederalkyl, gegebenenfalls durch Niederalkyl aza-substituiertes Azaniederalkylenaminoniederalkyl oder Pyridinioniederalkyl oder X Oxo oder Thioxo und $R_d$ Amino bedeuten, oder beide Reste $R_b$ und $R_c$ zusammengenommen Niederalkylen, Oxaniederalkylen, Thianiederalkylen oder gegebenenfalls durch Niederalkyl aza-substituiertes Azaniederalkylen bedeuten, und der andere der beiden Reste $R_3$ und $R_4$ dieselben Bedeutungen hat oder Oxo bedeutet und der Ring A keine weiteren Substituenten hat oder zusätzlich durch Niederalkyl, Niederalkoxy und/oder Halogen substituiert ist, sowie Salze von solchen Verbindungen mit salzbildenen Eigenschaften.

2. Verbindungen der Formel I gemäss Anspruch 1, worin $R_1$ Wasserstoff oder Niederalkyl, $R_2$ Wasserstoff, Niederalkyl oder Phenyl bedeutet, einer der Reste $R_3$ und $R_4$ die Gruppe =N-R (Ia) darstellt, worin R die Gruppe $-O-R_a$ (Ib) und $R_a$ Wasserstoff, Niederalkyl, Carboxyniederalkyl oder Niederalkoxycarbonylniederalkyl oder R die Gruppe $-N(R_b)-R_c$ (Ic) und $R_b$ und $R_c$ unabhängig voneinander Wasserstoff oder Niederalkyl oder einer der Reste $R_b$ und $R_c$ Wasserstoff und der andere Rest die Gruppe $-C(=X)-R_d$ darstellt, worin X Oxo und $R_d$ Niederalkoxy, Diniederalkylaminoniederalkyl, Triniederalkylammonioniederalkyl, Niederalkylenaminoniederalkyl, 4-Morpholinoniederalkyl, 4-Niederalkyl-1-piperazinoniederalkyl oder Pyridinioniederalkyl oder X Oxo oder Thioxo und $R_d$ Amino bedeuten, oder beide Reste $R_b$ und $R_c$ zusammengenommen Niederalkylen, 3-Oxa-1,5-pentylen oder 3-Niederalkyl-3-aza-1,5-pentylen bedeuten, und der andere der beiden Reste $R_3$ und $R_4$ für Oxo steht, und der Ring A keine weiteren Substituenten hat oder zusätzlich durch Niederalkyl, Niederalkoxy oder Halogen substituiert ist, wobei mit "nieder" bezeichnete Reste 1 oder 2 Kohlenstoffatome, Niederalkylenreste 4 oder 5 Kohlenstoffatome enthalten, und Halogen eine Atomnummer von höchstens 35 hat, und pharmazeutisch verwendbare Salze von solchen Verbindungen mit salzbildenden Eigenschaften.

3. Verbindungen der Formel I gemäss Anspruch 1, worin $R_1$ für Wasserstoff oder Niederalkyl und $R_2$ für Niederalkyl oder Phenyl stehen, $R_3$ eine Gruppe der Formel =N-R (Ia) darstellt, worin R die Gruppe $-O-R_a$ (Ib) und $R_a$ Niederalkyl, Carboxyniederalkyl oder Niederalkoxycarbonylniederalkyl oder R die Gruppe $-N(R_b)-R_c$ (Ic), eine der Reste $R_b$ und $R_c$ Wasserstoff und der andere Rest die Gruppe $-C(=X)-R_d$ darstellt, worin X Oxo oder Thioxo und $R_d$ Amino bedeuten oder beide Reste $R_b$ und $R_c$ zusammengenommen Niederalkylen, 3-Oxa-1,5-pentylen oder 3-Niederalkyl-3-aza-1,5-pentylen bedeuten, und $R_4$ für Oxo steht und der Ring A keine weiteren Substituenten hat, wobei mit "nieder" bezeichnete Reste 1 oder 2 Kohlentoffatome, Niederalkylenreste 4 oder 5 Kohlenstoffatome enthalten, und pharmazeutisch verwendbare Salze von solchen Verbindungen mit salzbildenden Eigenschaften.

4. Verbindungen der Formel I gemäss Anspruch 1, worin $R_1$ für Wasserstoff oder Methyl und $R_2$ für Methyl oder Phenyl stehen, $R_3$ die Gruppe =N-R (Ia) und R die Gruppe $-N(R_b)-R_c$ (Ic) darstellt, wobei einer der Reste $R_b$ und $R_c$ Wasserstoff und der andere Rest die Gruppe $-C(=X)-R_d$ (Id) bedeutet, worin X für Oxo und $R_d$ für Amino stehen, wobei mit "nieder" bezeichnete Reste in erster Linie ein, ferner auch zwei Kohlenstoffatome enthalten.

5. 2,2-Dimethyl-3,4-dihydro-2H-naphtho[1,2-b]pyran-5,6-dion-6-semicarbazon gemäss Anspruch 1.

6. 2,2-Dimethyl-3,4-dihydro-2H-naphtho[1,2-b]pyran-5,6-dion-6-thiosemicarbazon gemäss Anspruch 1.

7. 2,2-Dimethyl-3,4-dihydro-2H-naphtho[1,2-b]pyran-5,6-dion-6-(2-dimethylaminoacetylhydrazon) und Salze davon gemäss Anspruch 1.

8. 2,2-Dimethyl-3,4-dihydro-2H-naphtho[1,2-b]pyran-5,6-dion-6-(2-trimethylammonioacetylhydrazon) gemäss Anspruch 1.

9. 2,2-Dimethyl-3,4-dihydro-2H-naphtho[1,2-b]pyran-5,6-dion-6-(2-pyridinioacetylhydrazon) gemäss Anspruch 1.

10. Pharmazeutische Präparate enthaltend Verbindungen der Formel I oder pharmazeutisch verwendbare Salze von Verbindungen der Formel I mit salzbildenden Gruppen.

11. Verbindungen der Formel I oder pharmazeutisch verwendbare Salze von Verbindungen der Formel I zur Anwendung in einem Verfahren zur therapeutischen Behandlung des menschlichen oder tierischen Körpers.

12. Verbindungen der Formel I oder pharmazeutisch verwendbare Salze von Verbindungen der Formel I zur Hemmung der reversen Transkriptase.

13. Verfahren zur Herstellung von Verbindungen der Formel I und Salzen davon, dadurch gekennzeichnet, dass man eine Verbindung der Formel

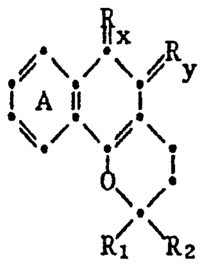

(II),

worin eine der Gruppen $R_x$ und $R_y$ für Oxo steht und die andere Oxo oder die Gruppe =N-R (Ia) darstellt, und $R_1$ und $R_2$ sowie der Ring A in der Formel II und R in der Gruppe Ia die oben gegebenen Bedeutungen haben, mit einer Verbindung der Formel $H_2N$-R (III), worin R die oben gegebene Bedeutung hat, umsetzt und, wenn erwünscht, eine erhaltene Verbindung in eine andere erfindungsgemässe Verbindung überführt und/oder, wenn erwünscht, ein erhaltenes Salz in die freie Verbindung oder in ein anderes Salz überführt oder eine erhaltene freie Verbidung mit salzbildenden Gruppen in ein Salz überführt.

14. Verwendung von Verbindungen der Formel I und pharmazeutisch verwendbaren Salze von Verbindungen der Formel I zur Herstellung von pharmazeutischen Präparaten.

FO 7.4 RS/cw*

<u>Ansprüche</u> (für den Vertragsstaat Oesterreich)

1. Verfahren zur Herstellung von Verbindungen der Formel I

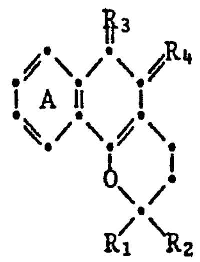

(I),

worin $R_1$ und $R_2$ unabhängig voneinander Wasserstoff, Niederalkyl oder Aryl oder zusammengenommen Niederalkylen bedeuten, einer der Reste $R_3$ und $R_4$ die Gruppe =N-R (Ia) darstellt, worin R die Gruppe $-O-R_a$ (Ib) und $R_a$ Wasserstoff, Niederalkyl, Carboxyniederalkyl oder Niederalkoxycarbonylniederalkyl oder R die Gruppe $-N(R_b)-R_c$ (Ic) und $R_b$ und $R_c$ unabhängig voneinander Wasserstoff oder Niederalkyl oder einer der Reste $R_b$ und $R_c$ Wasserstoff und der andere Rest die Gruppe $-C(=X)-R_d$ darstellt, worin X Oxo und $R_d$ Niederalkoxy, Diniederalkylaminoniederalkyl, Triniederalkylammonioniederalkyl, Niederalkylenaminoniederalkyl, Oxaniederalkylenammonioniederalkyl, Thianiederalkylenaminoniederalkyl, gegebenenfalls durch Niederalkyl aza-substituiertes Azaniederalkylenaminoniederalkyl oder Pyridinioniederalkyl oder X Oxo oder Thioxo und $R_d$ Amino bedeuten, oder beide Reste $R_b$ und $R_c$ zusammengenommen Niederalkylen, Oxaniederalkylen, Thianiederalkylen oder gegebenenfalls durch Niederalkyl aza-substituiertes Azaniederalkylen bedeuten, und der andere der beiden Reste $R_3$ und $R_4$ dieselben Bedeutungen hat oder Oxo bedeutet und der Ring A keine weiteren Substituenten hat oder zusätzlich durch Niederalkyl, Niederalkoxy und/oder Halogen substituiert ist, sowie Salzen von solchen Verbindungen mit salzbildenen Eigenschaften, dadurch gekennzeichnet, dass man eine Verbindung der Formel

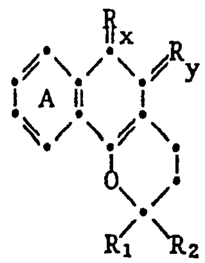

(II),

worin eine der Gruppen $R_x$ und $R_y$ für Oxo steht und die andere Oxo oder die Gruppe =N-R (Ia) darstellt, und $R_1$ und $R_2$ sowie der Ring A in der Formel II und R in der Gruppe Ia die oben gegebenen Bedeutungen haben, mit einer Verbindung der Formel $H_2N$-R (III), worin R die oben gegebene Bedeutung hat, umsetzt und, wenn erwünscht, eine erhaltene Verbindung in eine andere erfindungsgemässe Verbindung überführt und/oder, wenn erwünscht, ein erhaltenes Salz in die freie Verbindung oder in ein anderes Salz überführt oder eine erhaltene freie Verbidung mit salzbildenden Gruppen in ein Salz überführt.

2. Verfahren gemäss Anspruch 1 zur Herstellung von Verbindungen der Formel I, worin $R_1$ Wasserstoff oder Niederalkyl, $R_2$ Wasserstoff, Niederalkyl oder Phenyl bedeutet, einer der Reste $R_3$ und $R_4$ die Gruppe =N-R (Ia) darstellt, worin R die Gruppe $-O-R_a$ (Ib) und $R_a$ Wasserstoff, Niederalkyl, Carboxyniederalkyl oder Niederalkoxycarbonylniederalkyl oder R die Gruppe $-N(R_b)-R_c$ (Ic) und $R_b$ und $R_c$ unabhängig voneinander Wasserstoff oder Niederalkyl oder einer der Reste $R_b$ und $R_c$ Wasserstoff und der andere Rest die Gruppe $-C(=X)-R_d$ darstellt, worin X Oxo und $R_d$ Niederalkoxy, Diniederalkylaminoniederalkyl, Triniederalkylammonioniederalkyl, Niederalkylenaminoniederalkyl, 4-Morpholinoniederalkyl, 4-Niederalkyl-1-piperazinoniederalkyl oder Pyridinioniederalkyl oder X Oxo oder Thioxo und $R_d$ Amino bedeuten, oder beide Reste $R_b$ und $R_c$ zusammengenommen Niederalkylen, 3-Oxa-1,5-pentylen oder 3-Niederalkyl-3-aza-1,5-pentylen bedeuten, und der andere der beiden Reste $R_3$ und $R_4$ für Oxo steht, und der Ring A keine weiteren Substituenten hat oder zusätzlich durch Niederalkyl, Niederalkoxy oder Halogen substituiert ist, wobei mit "nieder" bezeichnete Reste 1 oder 2 Kohlenstoffatome, Niederalkylenreste 4 oder 5 Kohlenstoffatome enthalten, und Halogen eine Atomnummer von höchstens 35 hat, und pharmazeutisch verwendbaren Salzen von solchen Verbindungen mit salzbildenden Eigenschaften, dadurch gekennzeichnet, dass man eine Verbindung (II), worin $R_x$ und $R_y$ Oxo bedeuten, mit einer ensprechend substituierten Verbindung (III) umsetzt.

3. Verfahren gemäss Anspruch 1 zur Herstellung von Verbindungen der Formel I, worin $R_1$ für Wasserstoff oder Niederalkyl und $R_2$ für Niederalkyl oder Phenyl stehen, $R_3$ eine Gruppe der Formel =N-R (Ia) darstellt, worin R die Gruppe $-O-R_a$ (Ib) und $R_a$ Niederalkyl, Carboxyniederalkyl oder Niederalkoxycarbonylniederalkyl oder R die Gruppe $-N(R_b)-R_c$ (Ic) eine der Reste $R_b$ und $R_c$ Wasserstoff und der andere Rest die Gruppe $-C(=X)-R_d$ darstellt, worin X Oxo und Thioxo und $R_d$ Amino bedeuten oder beide Reste $R_b$ und $R_c$ zusammengenommen Niederalkylen, 3-Oxa-1,5-pentylen oder 3-Niederalkyl-3-aza-1,5-pentylen bedeuten, und $R_4$ für Oxo steht, wobei mit "nieder" bezeichnete Reste 1 oder 2 Kohlenstoffatome, Niederalkylenreste 4 oder 5 Kohlenstoffatome enthalten, und pharmazeutisch verwendbaren Salzen von solchen Verbindungen mit salzbildenden Eigenschaften, dadurch gekennzeichnet, dass man eine Verbindung (II), worin $R_x$ und $R_y$ Oxo bedeuten, mit einer ensprechend substituierten Verbindung (III) umsetzt.

4. Verfahren gemäss Anspruch 1 zur Herstellung von Verbindungen der Formel I, worin $R_1$ für Wasserstoff oder Methyl, und $R_2$ für Methyl oder Phenyl stehen, $R_3$ die Gruppe =N-R (Ia) und R die Gruppe $-N(R_b)-R_c$ (Ic) darstellt, wobei einer der Reste $R_b$ und $R_c$ Wasserstoff und der andere Rest die Gruppe $-C(=X)-R_d$ (Id) bedeutet, worin X für Oxo und $R_d$ für Amino stehen, wobei mit "nieder" bezeichnete Reste in erster Linie ein, ferner auch zwei Kohlenstoffatome enthalten, dadurch gekennzeichnet, dass man eine Verbindung (II), worin $R_x$ und $R_y$ Oxo bedeuten, mit einer ensprechend substituierten Verbindung (III) umsetzt.

5. Verfahren zur Herstellung von 2,2-Dimethyl-3,4-dihydro-2H-naphtho[1,2-b]pyran-5,6-dion-6-semicarbazon gemäss Anspruch 1, dadurch gekennzeichnet, dass man β-Lapachon mit Semicarbazid-hydrochlorid umsetzt.

6. Verfahren zur Herstellung von 2,2-Dimethyl-3,4-dihydro-2H-naphtho[1,2-b]pyran-5,6-dion-6-thiosemicarbazon gemäss Anspruch 1, dadurch gekennzeichnet, dass man β-Lapachon mit Thiosemicarbazid umsetzt.

7. Verfahren zur Herstellung von 2,2-Dimethyl-3,4-dihydro-2H-naphtho[1,2-b]pyran-5,6-dion-6-(2-dimethylaminoacetylhydrazon) und Salzen davon gemäss Anspruch 1, dadurch gekennzeichnet, dass man β-Lapachon mit N,N-Dimethylglycinhydrazid umsetzt.

8. Verfahren zur Herstellung von 2,2-Dimethyl-3,4-dihydro-2H-naphtho[1,2-b]pyran-5,6-dion-6-(2-trimethylammonioacetylhydrazon) gemäss Anspruch 1, dadurch gekennzeichnet, dass man β-Lapachon mit Trimethylammonioacetylhydrazidhydrochlorid umsetzt.

9. Verfahren zur Herstellung von 2,2-Dimethyl-3,4-dihydro-2H-naphtho[1,2-b]pyran-5,6-dion-6-(2-pyridinioacetylhydrazon) gemäss Anspruch 1, dadurch gekennzeichnet, dass man β-Lapachon mit 1-(2-Hydrazino-2-oxoäthyl)-pyridiniochlorid umsetzt.

FO 7.4/RS/cw*

0183649

Europäisches Patentamt

# EUROPÄISCHER RECHERCHENBERICHT

Nummer der Anmeldung

EP 85810555.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | GB - A - 1 040 176 (MERCK & CO)<br>* Anspruch 1 *<br>-- | 1,10,11,<br>13 | C 07 D 311/92<br>A 61 K 31/35 |
| A | AT - B - 334 896 (CARLO ERBA S.P.A.)<br>* Seite 2, Zeilen 1-14 *<br>-- | 1,10,11<br>13 | |
| A | FR - A1 - 2 372 822 (HOECHST AKTIENGESELLSCHAFT)<br>* Anspruch 1 *<br>---- | 1,10,11,<br>13 | |

| RECHERCHIERTE SACHGEBIETE (Int Cl 4) |
|---|
| C 07 D 311/00 |

Der vorliegende Recherchenbericht wurde für alle Patentansprüche erstellt.

| Recherchenort | Abschlußdatum der Recherche | Prüfer |
|---|---|---|
| WIEN | 28-02-1986 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82